# EUROPEAN PATENT SPECIFICATION

(11) **EP 2 025 310 B1**
(45) Date of publication and mention of the grant of the patent: **09.11.2011**
(21) Application number: 08012942.2
(22) Date of filing: 17.07.2008
(51) Int. Cl.: A61F 13/00

(54) **Patch and adhesive preparation**
Pflaster und Haftvorrichtung dafür
Pansement et préparation adhésive

(30) Priority: 20.07.2007 JP 2007189250
(43) Date of publication of application: 18.02.2009
(73) Proprietor: Nitto Denko Corporation, Ibaraki-shi Osaka 567-8680 (JP)
(72) Inventor: Hashino, Ryo, Ibaraki-shi Osaka 567-8680 (JP); Konno, Masakatsu, Ibaraki-shi Osaka 567-8680 (JP); Harima, Jun, Ibaraki-shi Osaka 567-8680 (JP)
(74) Representative: Grünecker, Kinkeldey, Stockmair & Schwanhäusser

(56) References cited:
- EP-A- 0 356 614
- EP-A- 0 566 816
- WO-A-90/01915
- US-A- 5 733 251
- US-B1- 7 115 276

## Description

### FIELD OF THE INVENTION

The present invention relates to a patch and an adhesive preparation.

### BACKGROUND OF THE INVENTION

Various patches and adhesive preparations have hitherto been developed for the purposes of, e.g., protecting the skin or administering a drug to the living body through the skin. Especially, in recent years, soft pressure-sensitive adhesive layers obtained by incorporating an organic liquid ingredient into pressure-sensitive adhesive layers have been developed in order to reduce the physical stimulus to be given to the skin upon stripping or enable the patch to give a soft wear feeling. Furthermore, There are cases where a thick pressure-sensitive adhesive layer is employed for the purpose of enabling the pressure-sensitive adhesive layer to hold a large amount of a drug dissolved therein or for another purpose.

However, since the pressure-sensitive adhesive layer of such a patch is soft or thick, there are cases where the pressure-sensitive adhesive layer protrudes from an edge of the patch or flows out during patch storage due to the so-called cold-flow phenomenon. The pressure-sensitive adhesive which has protruded or flowed out may adhere to an inner surface of the package, making it difficult to take the patch out of the package, or the user of this patch may come to have a sticky hand and an uncomfortable feeling. The so-called dry edge is known as a technique for avoiding such a phenomenon.

JP-A-11-1432 discloses a patch having the so-called dry edge in which the release liner is projected from the edges of the backing and pressure-sensitive adhesive layer. According to this patch, the pressure-sensitive adhesive layer is inhibited in some degree from protruding or flowing out from that edge of the pressure-sensitive adhesive layer which is in contact with the release liner. However, such a patch has a possibility that the pressure-sensitive adhesive layer might protrude or flow out from that edge of the pressure-sensitive adhesive layer which is in contact with the backing, and therefore this patch is not fully satisfactory.

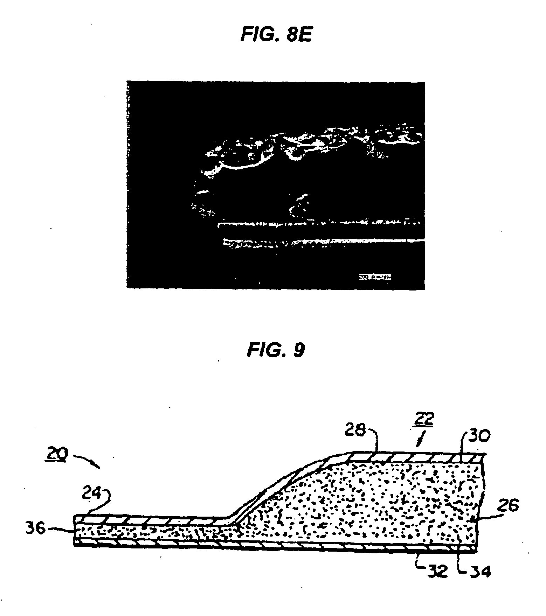

JP-A-06-063071 discloses a dressing for wounds which has a pressure-sensitive adhesive layer contoured so as to fit the skin. One embodiment of this dressing is shown in Fig. 9. Since this dressing has a cut edge where the constituent layers are flush with each other, there is a possibility that the pressure-sensitive adhesive layer might protrude or flow out from the edge and adhere to an inner surface of the package.

Another embodiment of the dressing disclosed in this document is shown in Fig. 10. In this embodiment, the edge of the pressure-sensitive adhesive layer is covered with the backing. However, since an edge part of the backing covers the edge of the pressure-sensitive adhesive layer and the edge of the backing coincides with the edge of the release liner in the edge part of this patch, there is a possibility that it might be difficult for the user to pinch the edge of the release liner with fingers to peel off the release liner. In addition, since the edge part of the backing is required to retain a bent shape for covering the edge of the pressure-sensitive adhesive layer, this patch has a low degree of freedom of the selection of backing materials and shapes thereof, in particular, thickness, etc. Especially, when the pressure-sensitive adhesive layer is thick, it may be practically difficult to bend an edge part of the backing so as to cover the whole edge of the pressure-sensitive adhesive layer. Actually, the pressure-sensitive adhesive layer of the patch in this embodiment is formed so as to have an edge-part thickness smaller than the thickness thereof in a central part.

WO 90/01915 A describes an adhesive dressing, wherein, when using the dressing, a protective backing layer has to be removed, then a first film layer is adhered to the skin and, after that, a second film layer is removed. The first film is coated with an adhesive and the edges thereof are cut in a conventional manner.

EP 0 356 614 A2 discloses a dermal applicator comprising a film layer and an adhesive corresponding to the backing and the adhesive layer of the invention, respectively. EP 0 566 816 A1 describes a pouch patch application with a film layer and an adhesive layer corresponding to the backing and the adhesive layer of the invention, respectively. US 5 733 251 A describes a medical dressing comprising a dressing layer and an adhesive corresponding to the backing and the adhesive layer of the invention, respectively. However, the edges of the film/dressing layers and adhesive layers in each of the these documents are cut in a conventional manner.

US 7 115 276 B1 describes a self-adhering plaster having a multi-layer construction, wherein an adhesive core is surrounded by an adhesive ring with reduced flowability in order to reduce cold flow.

### SUMMARY OF THE INVENTION

In view of the above, an object of the invention is to provide a patch and an adhesive preparation in each of which the pressure-sensitive adhesive is less apt to protrude or flow out from the edge parts thereof.

An object of the invention is surprisingly accomplished by regulating the pressure-sensitive adhesive layer in a patch so as to have a given sectional shape at an edge part thereof. Accordingly, the invention provides the following (1) to (7).
(1) A patch comprising the features defined in claim 1.
(2) The patch according to (1), wherein at least said part of the edge of the pressure-sensitive adhesive layer is located on the center side of the patch with respect to either the edge of the backing or an edge of the release liner.
(3) The patch according to (1) or (2), wherein the edge of the pressure-sensitive adhesive layer has such a sectional shape that the pressure-sensitive adhesive layer is bent toward the center of the patch.
(4) The patch according to any one of (1) to (3), wherein an edge of the release liner protrudes toward the edge part side of the patch from a position on the release liner where the perpendicular segment drawn from the edge of the backing to the release liner intersects the release liner.
(5) The patch according to any one of (1) to (4), wherein the pressure-sensitive adhesive layer contains an organic liquid component.
(6) The patch according to any one of (1) to (5), which is an adhesive preparation comprising a pressure-sensitive adhesive layer containing a drug.

According to the invention, since at least a part of the edge of the pressure-sensitive adhesive layer is located in a given position on the patch center side, the pressure-sensitive adhesive layer is inhibited from protruding from the edge part of the patch or flowing out during patch storage due to the so-called cold-flow phenomenon. The pressure-sensitive adhesive layer is hence inhibited from adhering to the inner surfaces of the package of the patch. Consequently, the patch can be easily taken out of the package and the user is less apt to come to have a sticky hand and an uncomfortable feeling.

Furthermore, in the patch of the invention, the edge of the pressure-sensitive adhesive layer is exposed. Namely, the edge of the pressure-sensitive adhesive layer is covered with neither an edge of the backing nor an edge of the release liner. Owing to this constitution as well as the above-mentioned constitution in which the pressure-sensitive adhesive layer is inhibited from protruding or flowing out and use of the patch is hence less apt to result in a sticky hand, it is extremely easy to pinch the edge of the release liner of the patch of the invention with fingers to peel off the release liner.

As described above, a patch which is smoothly usable can be provided according to the invention.

### BRIEF DESCRIPTION OF THE DRAWINGS

Fig. 1A is a diagrammatic slant view of and Fig. 1B is a diagrammatic sectional view of an embodiment of the patch of the invention.
Fig. 2 is a diagrammatic sectional view of another embodiment of the patch of the invention.
Fig. 3 is a diagrammatic sectional view of still another embodiment of the patch of the invention.
Fig. 4 is a diagrammatic sectional view of a further embodiment of the patch of the invention.
Fig. 5 is a diagrammatic sectional view of still a further embodiment of the patch of the invention.
Fig. 6 is a diagrammatic sectional view of the patch of a comparative embodiment.
Fig. 7 is a diagrammatic sectional view of the patch of another comparative embodiment.
Figs. 8A to 8C are photographs of sections of the patches of the Examples 1, 2 and 3 and Figs. 8D and 8E are photographs of sections of the patches of the Comparative Examples 1 and 2.
Fig. 9 is a sectional view of an embodiment of conventional patch.

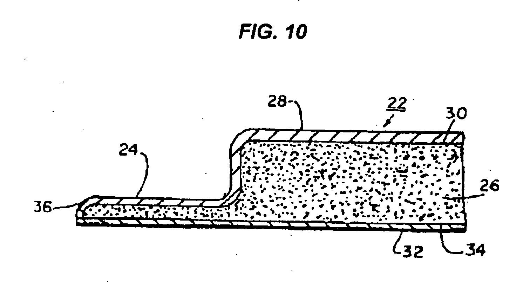

Fig. 10 is a sectional view of another embodiment of the conventional patch.

### Description of Reference Numerals

- 10: patch
- 1: pressure-sensitive adhesive layer
- 2: backing
- 3: release liner
- 4: part
- 5: segment
- 6: distance
- 7: distance
- 8: angle
- 9: angle
- 20: dressing
- 22: absorptive layer
- 24: flange layer
- 26: pressure-sensitive adhesive layer
- 28: backing
- 30: main surface
- 32: release liner
- 34: second main surface
- 36: edge

### DETAILED DESCRIPTION OF THE INVENTION

Preferred embodiments of the invention are shown below. However, the following detailed explanations thereon and specific examples are intended only for exemplification and should not limit the scope of the invention. The following explanations on preferred embodiments are merely illustrative and are never intended to limit the invention and the applications or uses thereof.

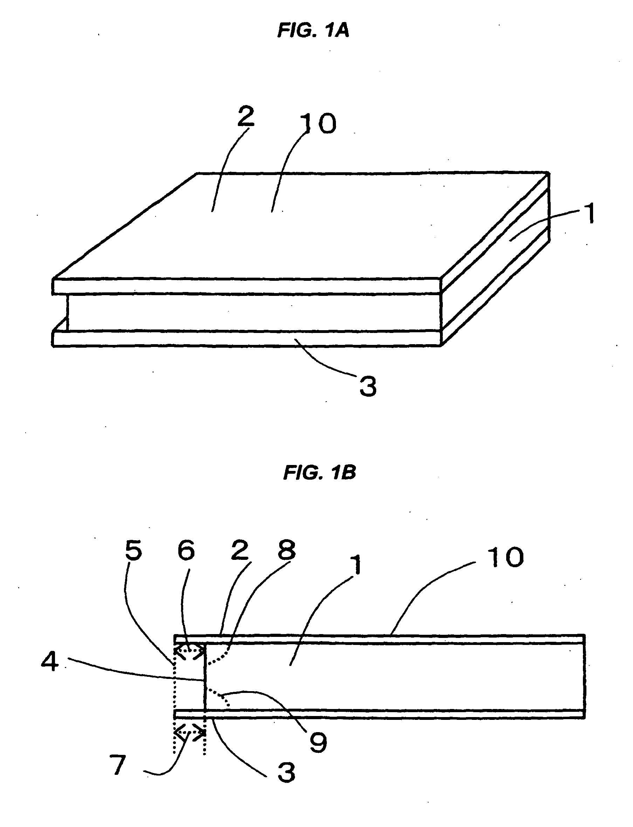

Fig. 1A is a slant view of a patch 10 of one embodiment of the invention. Fig. 1B is a sectional view of the patch. With reference to Fig. 1A, the patch 10 of the invention includes a pressure-sensitive adhesive layer 1, a backing 2, and a release liner 3. With reference to Fig. 1B, the patch 10 at an edge part thereof has such a sectional shape that, when a perpendicular segment 5 is drawn from an edge of the backing 2 at the edge part of the patch 10 to the release liner 3, then at least a part 4 of the edge of the pressure-sensitive adhesive layer 1 at the edge part of the patch 10 is located on the patch center side with respect to the segment 5.

With reference to Fig. 1A, although the shape of the patch of the invention is not particularly limited, the patch 10 of this embodiment has a substantially planar sheet form, and the planar shape thereof is substantially rectangular. Although the size thereof is not particularly limited, a specific example has a shape in which one side has a length of about 20-80 mm and another side has a length of about 20-80 mm. Other planar shapes such as substantially polygonal shapes including substantially triangular shapes and substantially pentagonal shapes, substantially elliptic shapes, substantially circular shapes, and other various shapes may also be employed.

The thickness of the pressure-sensitive adhesive layer is 10-700 µm, more preferably 50-600 µm, and most preferably 100-500 µm. In ordinary patches, when the pressure-sensitive adhesive layer is thick in some degree, i.e., has a thickness of 10 µm or larger, there is a higher tendency for the pressure-sensitive adhesive layer to protrude or flow out. The invention can effectively inhibit this phenomenon and is hence advantageous especially in such cases. On the other hand, in a case where the thickness of the pressure-sensitive adhesive layer exceeds 1,000 µm, there is a tendency that it is difficult for the pressure-sensitive adhesive layer to retain a given shape.

The thickness of the backing 2 is 10-200 µm, preferably 15-150 µm, and more preferably 20-100 µm. When the thickness thereof is 10 µm or larger, the pressure-sensitive adhesive layer which has protruded or flowed out from an edge of the pressure-sensitive adhesive layer is inhibited from moving to the surface of the backing which is opposite to the surface on which the pressure-sensitive adhesive layer is disposed. In a case where the thickness of the backing 2 exceeds 200 µm, there is a possibility that this patch might give a stiff feeling during wear.

The distance 6 between the edge of the backing 2 and the position where the edge of the pressure-sensitive adhesive layer 1 is in contact with the backing 2 is not particularly limited. However, the distance 6 is preferably 1-100 µm, more preferably 1-90 µm, and most preferably 2-80 µm. When the distance 6 is 1 µm or longer, the pressure-sensitive adhesive layer 1 is effectively inhibited from protruding from the edge of the pressure-sensitive adhesive layer 1 at which the pressure-sensitive adhesive layer 1 is in contact with the backing 2 or from flowing out from the edge during the storage of the patch 10. On the other hand, in a case where the distance 6 is longer than 100 µm, there is a possibility that the edge of the backing 2 might readily lift up from the applied surface during wear of the patch 10.

The distance 7 between the edge of the release liner 3 and the position where the edge of the pressure-sensitive adhesive layer 1 is in contact with the release liner 3 is not particularly limited. However, the distance 7 is preferably 1-100 µm, more preferably 1-90 µm, and most preferably 2-80 µm. When the distance 7 is 1 µm or longer, the pressure-sensitive adhesive layer 1 is effectively inhibited from protruding from the edge of the pressure-sensitive adhesive layer 1 at which the pressure-sensitive adhesive layer 1 is in contact with the release liner 3 or from flowing out from the edge during the storage of the patch 10. On the other hand, when the distance 7 is 100 µm or shorter, it is possible to reduce the size of the patch 10 to thereby achieve material saving.

At the position where the edge of the pressure-sensitive adhesive layer 1 is in contact with the backing 2, the angle 8 on the patch center side which is formed by a plane including the surface of the backing 2 facing the pressure-sensitive adhesive layer 1 and a contact plane of the edge of the pressure-sensitive adhesive layer 1 (in the embodiment shown in Figs. 1A and 1B, the contact plane coincides with the edge itself of the pressure-sensitive adhesive layer 1) is not particularly limited. However, in the embodiment shown in Figs. 1A and 1B, the angle 8 is nearly a right angle, e.g., 80-100 degrees. Further, at the position where the edge of the pressure-sensitive adhesive layer 1 is in contact with the release liner 3, the angle 9 on the patch center side which is formed by a plane including the surface of the release liner 3 facing the pressure-sensitive adhesive layer 1 and a contact plane of the edge of the pressure-sensitive adhesive layer 1 (in the embodiment shown in Figs. 1A and 1B, the contact plane coincides with the edge itself of the pressure-sensitive adhesive layer 1) may have the similar degrees as the angle 8.

Furthermore, in the invention, the edge of the pressure-sensitive adhesive layer 1 is exposed, i.e., it is not covered with an edge part of the backing 2 and/or an edge part of the release liner 3. In addition, at least a part 4 of the edge of the pressure-sensitive adhesive layer 1 in the edge part of the patch 10 is located at a given position. Accordingly, when the user pinches the edge of the release liner 3 with fingers to peel off the release liner 3, the fingers do not become excessively sticky and areas suitable for pinching are easily obtained. As a result, the release liner 3 can be easily peeled off.

In the patch shown in Figs. 1A and 1B, the edge of the pressure-sensitive adhesive layer 1 has a substantially linear sectional shape. However, the shape thereof should not be construed as being limited to linear ones. The sectional shape thereof may be, for example, a curved shape such as a curved shape protrudent toward the patch center side or edge side, a wavy shape, a zigzag shape, or the like.

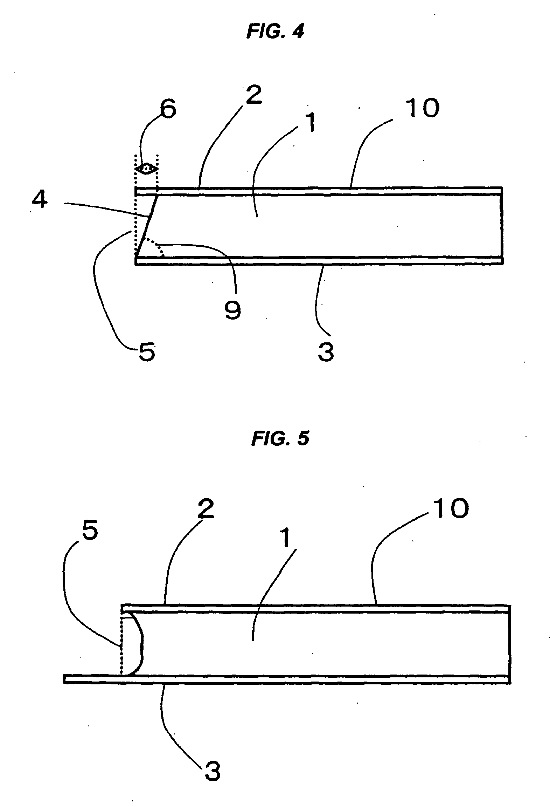

According to necessity, the edge of the release liner 3 may be located so as to protrude toward the edge part side of the patch 10 from a position on the release liner 3 where a perpendicular segment 5 drawn from the edge of the backing 2 to the release liner 3 intersects the release liner 3. The effects of this constitution will be described later with reference to Fig. 5.

The patch shown in Figs. 1A and 1B may be produced, for example, by the following method. A near-edge part of the release liner of a patch produced in an ordinary manner is once peeled off, and an edge part of the pressure-sensitive adhesive layer is partly removed with a cutter or the like while taking care not to damage the backing. Thereafter, the release liner is returned to the original position to produce the objective patch.

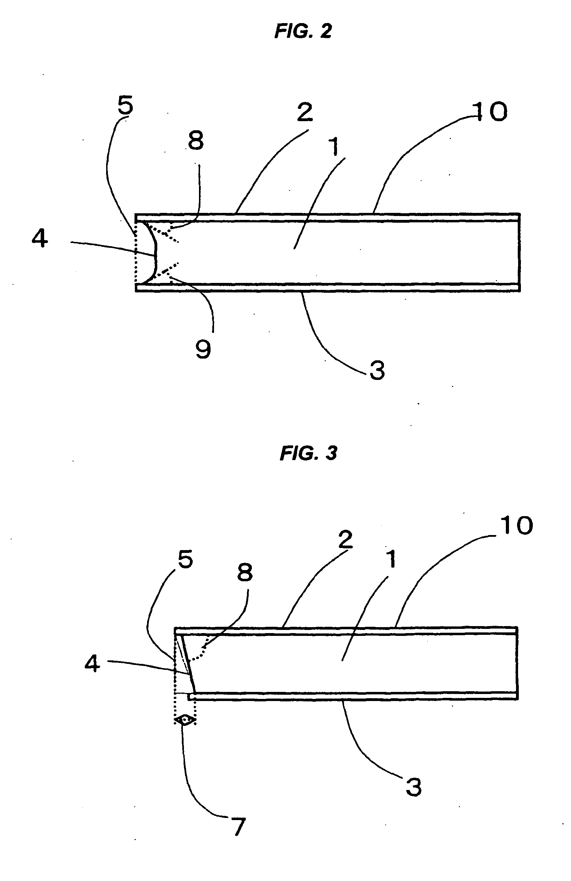

In the next place, with reference to Fig. 2, Fig. 2 is a sectional view of a patch 10 of another embodiment of the invention. Also in this embodiment, the patch 10 at an edge part thereof has such a sectional shape that, when a perpendicular segment 5 is drawn from the edge of the backing 2 at the edge part of the patch 10 to the release liner 3, then at least a part 4 of the edge of the pressure-sensitive adhesive layer 1 at the edge part of the patch 10 is located on the patch center side with respect to the segment 5.

In this embodiment, the edge of the pressure-sensitive adhesive layer 1 has such a sectional shape that the pressure-sensitive adhesive layer 1 is bent toward the center side of the patch 10. Namely, this patch 10 at an edge part thereof has a sectional shape in which at least a part 4 (e.g., a nearly central part in the thickness direction of the patch 10) of the edge of the pressure-sensitive adhesive layer 1 at the edge part of the patch 10 is located on the center side of the patch 10 with respect to a segment which connects the position where the edge of the pressure-sensitive adhesive layer 1 is in contact with the backing 2 to the position where the edge of the pressure-sensitive adhesive layer 1 is in contact with the release liner 3, whereby the part 4 is separated from the segment at a certain distance. This distance is preferably 10 µm or longer, more preferably 20 µm or longer, as measured in the direction parallel to the patch backing or release liner. Unexpectedly, when the pressure-sensitive adhesive layer 1 is prepared so as to have such a shape, the pressure-sensitive adhesive layer 1 is especially effectively inhibited from protruding or flowing out. The longer this distance is, the higher the effect is. However, this distance is preferably 200 µm or shorter from the standpoint of ease of production.

Furthermore, in the embodiment shown in Fig. 2, the pressure-sensitive adhesive layer 1 is sufficiently inhibited from protruding or flowing out even when the position of the edge of the backing 2 substantially coincides with the position where the edge of the pressure-sensitive adhesive layer 1 is in contact with the backing 2 (i.e., even when the distance corresponding to the distance 6 in the embodiment shown in Figs. 1A and 1B is about 0-25 µm). Likewise, in this embodiment, the pressure-sensitive adhesive layer 1 is sufficiently inhibited from protruding or flowing out even when the position of the edge of the release liner 3 substantially coincides with the position where the edge of the pressure-sensitive adhesive layer 1 is in contact with the release liner 3 (i.e., even when the distance corresponding to the distance 7 in the embodiment shown in Figs. 1A and 1B is about 0-25 µm). These effects are attained when either of or preferably both of the angle 8 formed by a plane including the surface of the backing 2 facing the pressure-sensitive adhesive layer 1 and a contact plane of the edge of the pressure-sensitive adhesive layer 1 as well as the angle 9 formed by a plane including the surface of the release liner 3 facing the pressure-sensitive adhesive layer 1 and a contact plane of the edge of the pressure-sensitive adhesive layer 1 are an acute angle.

Constitutions of the embodiment shown in Fig. 2, which are not especially described herein, are the same as those of the embodiment shown in Figs. 1A and 1B.

The patch shown as an embodiment in Fig. 2 is produced, for example, by the following method. At an edge part of a patch, a cutter is applied to the patch from the surface of the backing at a given angle and the backing and the pressure-sensitive adhesive layer are cut to a nearly central part thereof, followed by removing a part of the backing and a part of the pressure-sensitive adhesive layer. Thereafter, at the edge part of the patch, a cutter is applied to the patch from the surface of the release liner at a given angle and the release liner and the pressure-sensitive adhesive layer are cut to a nearly central part thereof to remove a part of the release liner and a part of the pressure-sensitive adhesive layer.

In the next place, with reference to Fig. 3, Fig. 3 is a sectional view of a patch 10 of still another embodiment of the invention. Also in this embodiment, the patch 10 at an edge part thereof has such a sectional shape that, when a perpendicular segment 5 is drawn from the edge of the backing 2 at the edge part of the patch 10 to the release liner 3, then at least a part 4 of the edge of the pressure-sensitive adhesive layer 1 at the edge part of the patch 10 is located on the patch center side with respect to the segment 5.

In the sectional view of this embodiment, the position of the edge of the backing 2 substantially coincides with the position where the edge of the pressure-sensitive adhesive layer 1 is in contact with the backing 2. In this embodiment, the distance corresponding to the distance 6 in the embodiment shown in Figs. 1A and 1B is about 0-25 µm. However, the pressure-sensitive adhesive layer at the edge part of the patch 10 is inhibited from protruding or flowing out from the backing 2 side. This effect is attained when the angle 8 on the patch central side which is formed by a plane including the surface of the backing 2 facing the pressure-sensitive adhesive layer 1 and a contact plane of the edge of the pressure-sensitive adhesive layer 1 is an acute angle, preferably 80 degrees or smaller. Incidentally, as mentioned later with reference to Fig. 5, the inhibition of the protrusion or outflow of the pressure-sensitive adhesive layer 1 from the release liner 3 side can be attained when the edge of the release liner 3 protrudes toward the edge part side of the patch 10 from the position on the release liner 3 where a perpendicular segment 5 drawn from the edge of the backing 2 to the release liner 3 intersects the release liner 3.

Although the sectional shape of the edge of the pressure-sensitive adhesive layer 1 is represented by the substantially linear segment connecting both ends thereof, the sectional shape thereof is not limited thereto. Constitutions of the embodiment shown in Fig. 3, which are not especially described herein, are the same as those of the embodiment shown in Figs. 1A and 1B.

The patch shown as an embodiment in Fig. 3 is produced, for example, by the following method. At an edge part of a patch, a cutter is applied to the patch from the surface of the backing at a given angle and the backing and the pressure-sensitive adhesive layer are cut, followed by removing the resultant edge-side fragment of the backing and pressure-sensitive adhesive layer. It should, however, be noted that the release liner is not cut.

In the next place, with reference to Fig. 4, Fig. 4 is a sectional view of a patch 10 of a further embodiment of the invention. Also in this embodiment, the patch 10 at an edge part thereof has such a sectional shape that, when a perpendicular segment 5 is drawn from the edge of the backing 2 at the edge part of the patch 10 to the release liner 3, then at least a part 4 of the edge of the pressure-sensitive adhesive layer 1 at the edge part of the patch 10 is located on the patch center side with respect to the segment 5.

In the sectional view of this embodiment, the edge of the backing 2 and the position where the edge of the pressure-sensitive adhesive layer 1 is in contact with the backing 2 provide a certain distance 6. Owing to the distance 6, the pressure-sensitive adhesive layer at the edge part of the patch 10 is inhibited from protruding or flowing out from the backing 2 side. The distance 6 is the same as in the embodiment shown in Figs. 1A and 1B.

On the other hand, the position of the edge of the release liner 3 substantially coincides with the position on the release liner 3 where a perpendicular segment 5 drawn from the edge of the backing 2 to the release liner 3 intersects with the release liner 3. In this embodiment, the distance corresponding to the distance 7 in the embodiment shown in Figs. 1A and 1B is about 0-25 µm. In this embodiment, the pressure-sensitive adhesive layer 1 is effectively inhibited from protruding or flowing out from the release liner 3 side. This effect is attained when the angle 9 on the patch center side which is formed by a plane including the surface of the release liner 3 facing the pressure-sensitive adhesive layer 1 and a contact plane of the edge of the pressure-sensitive adhesive layer 1 is an acute angle, preferably 80 degrees or smaller. Although the sectional shape of the edge of the pressure-sensitive adhesive layer 1 is represented by the substantially linear segment connecting both ends thereof, the sectional shape thereof is not limited thereto. Constitutions of the embodiment shown in Fig. 4 which are not especially described are the same as those of the embodiment shown in Figs. 1A and 1B.

The patch shown as an embodiment in Fig. 4 is produced, for example, by the following method. At an edge part of a patch, a cutter is applied to the patch from the surface of the release liner at a given angle and the release liner and the pressure-sensitive adhesive layer are cut, followed by removing the resultant edge-side fragment of the release liner and pressure-sensitive adhesive layer. It should, however, be noted that the backing is not cut.

In the next place, with reference to Fig. 5, Fig. 5 is a sectional view of a patch 10 of still a further embodiment of the invention. This embodiment is a modification of the embodiment of the invention shown in Fig. 2. Specifically, in this patch embodiment, the edge of the release liner 3 protrudes toward the edge part side of the patch 10 from the position on the release liner 3 where a perpendicular segment 5 drawn from the edge of the backing 2 to the release liner 3 intersects the release liner 3. Besides the effect described above with reference to Fig. 2, this embodiment is more effective especially in inhibiting the pressure-sensitive adhesive layer 1 from protruding or flowing out from the release liner 3 side because the edge of the release liner 3 protrudes as shown above.

The distance between the edge of the release liner 3 and the position on the release liner where the perpendicular segment 5 drawn from the edge of the backing 2 to the release liner 3 intersects the release liner 3 is not particularly limited. However, the distance is preferably 0.5-10 mm, and more preferably 1-8 mm. When the distance is 0.5 mm or longer, the pressure-sensitive adhesive layer 1 is effectively inhibited from protruding or flowing out. When the distance is 10 mm or shorter, the patch 10 as a whole is inhibited from increasing in size and the release liner 3 is inhibited from causing an increased material waste.

Constitutions of the embodiment shown in Fig. 5, which are not especially described herein, are the same as those of the embodiment shown in Figs. 1A and 1B. Although the embodiment shown in Fig. 5 is a modification of the embodiment shown in Fig. 2, the same modification is also possible in the embodiments shown in Figs. 1, 3, and 4. Such modified patches can be produced by the same production methods as those described above with regard to Figs. 1A to 4.

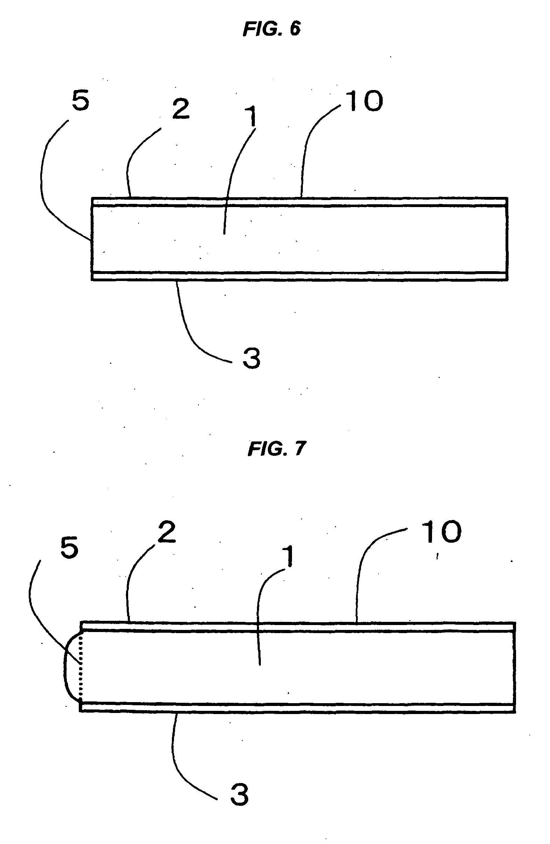

Fig. 6 is a sectional view of an embodiment of conventional patches 10. In this embodiment, the patch 10 has such a sectional shape that the edge of the pressure-sensitive adhesive layer 1 is located on a perpendicular segment 5 drawn from the edge of the backing 2 to the release liner 3. Consequently, the pressure-sensitive adhesive layer 1 is apt to protrude or flow out.

Fig. 7 is a sectional view of another embodiment of conventional patches 10. In this embodiment, the patch 10 has such a sectional shape that the edge of the pressure-sensitive adhesive layer 1 is located on the edge side of the patch 10 with respect to a perpendicular segment 5 drawn from the edge of the backing 2 to the release liner 3. Consequently, the pressure-sensitive adhesive layer 1 is apt to protrude or flow out. Incidentally, the evaluation values concerning the sectional shapes of patches in this specification mean values obtained by the methods which will be described in Examples.

In the patch of the invention described above, the backing is not particularly limited, and a known material in a film or sheet form may be used. As such a backing, it is preferable to use those which are substantially impermeable to components of the pressure-sensitive adhesive layer, such as a drug and additives, and prevents the components from passing therethrough and evaporating off from the back to cause a decrease in content.

Examples of the backing include single-layer films of polyesters, nylons, Saran (registered trademark), polyethylene, polypropylene, poly(vinyl chloride), ethylene/ethyl acrylate copolymers, polytetrafluoroethylene, Surlyn (registered trademark), and metal foils and laminated films composed of two or more thereof.

The pressure-sensitive adhesive layer is not particularly limited. Examples of the pressure-sensitive adhesive include acrylic pressure-sensitive adhesives containing an acrylic polymer; rubber pressure-sensitive adhesives such as styrene/diene/styrene block copolymers (e.g., styrene/isoprene/styrene block copolymers and styrene/butadiene/styrene block copolymers), polyisoprene, polyisobutylene, and polybutadiene; silicone pressure-sensitive adhesives such as silicone rubbers, dimethylsiloxane-based polymers, and diphenylsiloxane-based polymers; vinyl ether pressure-sensitive adhesives such as poly(vinyl methyl ether), poly(vinyl ethyl ether), and poly(vinyl isobutyl ether); vinyl ester pressure-sensitive adhesives such as vinyl acetate/ethylene copolymers; and polyester pressure-sensitive adhesives produced from a carboxylic acid ingredient such as dimethyl terephthalate, dimethyl isophthalate, or dimethyl phthalate and a polyhydric alcohol ingredient such as ethylene glycol. The pressure-sensitive adhesive layer may be crosslinked or non-crosslinked. From the standpoint of adhesion to the skin, hydrophobic pressure-sensitive adhesives are preferred and pressure-sensitive adhesive layers containing no water are preferred.

Of those pressure-sensitive adhesives, the rubber pressure-sensitive adhesives tend to give a pressure-sensitive adhesive layer which is apt to protrude or flow out. Since the invention can effectively inhibit such a protrusion or outflow, the invention is especially advantageous in the case where a rubber pressure-sensitive adhesive is used for the pressure-sensitive adhesive layer. For the same reason, the non-crosslinked pressure-sensitive adhesive layer is advantageous.

A mixture of rubber pressure-sensitive adhesives which are constituted of the same component or different components and differ from each other in average molecular weight can be used in order to obtain moderate adhesive force and drug solubility. For example, when polyisobutylenes are employed as an example, it is preferred to use a mixture of a high-molecular polyisobutylene having a viscosity average molecular weight of 1,800,000-5,500,000 with a medium-molecular polyisobutylene having a viscosity average molecular weight of 40,000-85,000 and optionally a low-molecular polyisobutylene having a lower viscosity average molecular weight.

In this case, it is preferred that the high-molecular polyisobutylene be incorporated in a proportion of 10-80% by weight, preferably 10-50% by weight, the medium-molecular polyisobutylene be incorporated in a proportion of 0-90% by weight, preferably 0-80% by weight, and the low-molecular polyisobutylene be incorporated in a proportion of 0-80% by weight, preferably 10-60% by weight. The term, average molecular weight herein means the viscosity-average molecular weight calculated with the Flory viscosity equation.

A tackifier such as a rosin resin, polyterpene resin, coumarone-indene resin, petroleum resin, terpene-phenol resin, or xylene resin may have been incorporated in rubber pressure-sensitive adhesives in order to impart moderate tackiness. Such tackifiers may be used alone or as a mixture of two or more thereof. The content of the tackifier in the pressure-sensitive adhesive layer is, for example, 10-40% by weight.

The pressure-sensitive adhesive layer may contain a drug according to the necessity, whereby an adhesive preparation can be provided. The drug used herein is not particularly limited. Preferred is a drug which can be administered to a mammal such as a human being through the skin, i.e., which is percutaneously absorbable.

The organic liquid component is not particularly limited. Examples thereof include glycols such as ethylene glycol, diethylene glycol, propylene glycol, dipropylene glycol, tripropylene glycol, triethylene glycol, polyethylene glycol, and polypropylene glycol; fats and oils such as olive oil, castor oil, and lanolin; hydrocarbons such as squalane and liquid paraffin; various surfactants; ethoxy stearyl alcohol; glycerol monoesters such as oleic acid monoglyceride, caprylic acid monoglyceride, and lauric acid monoglyceride, glycerol diesters such as polypropylene (in general, polyalkylene) glycol dialkyl esters, glycerol triesters such as glycerol triacetate, and mixtures thereof; alkyl esters of fatty acids, such as triethyl citrate; higher alcohols; higher fatty acids such as oleic acid and caprylic acid; pyrrolidone compounds such as N-methylpyrrolidone and N-dodecylpyrrolidone; sulfoxides such as decyl methyl sulfoxide; and 1,3-butanediol. These ingredients may be used alone or as a mixture of two or more thereof. The organic liquid component can be incorporated in an amount of preferably 10-60% by weight, more preferably 15-60% by weight, and most preferably 20-60% by weight, based on the total weight of the pressure-sensitive adhesive layer. When a pressure-sensitive adhesive layer contains such an organic liquid component in an amount of 10% by weight or larger, this pressure-sensitive adhesive layer is apt to be plasticized and to protrude or flow out. Since the invention can effectively inhibit this protrusion or outflow, the invention is advantageous in such a case. Incidentally, in a case where the organic liquid component is contained in an amount exceeding 60% by weight, there is a possibility that it is difficult for the pressure-sensitive adhesive layer to retain a given shape.

The release liner is not particularly limited. Examples of the material thereof include materials which are known in this field. Specific examples thereof include films of plastics such as polyesters including poly(ethylene terephthalate), poly(vinyl chloride), poly(vinylidene chloride), various acrylic and methacrylic polymers, polystyrene, polycarbonates, polyimides, cellulose acetate (acetate), regenerated cellulose (cellophane), and celluloid and laminated films composed of wood-free paper, glassine paper, or the like and a polyolefin. From the standpoints of safety, profitability, and drug migration prevention, it is preferred to use a polyester film. The release liner preferably is one in which the side facing the pressure-sensitive adhesive layer has undergone a releasant treatment so as to facilitate peeling from the pressure-sensitive adhesive layer.

### Examples

### (1) Preparation of Coating Fluid

The following ingredients were weighed out: 625.0 g of toluene, 875.0 g of n-hexane, 104.3 g of high-molecular polyisobutylene (viscosity-average molecular weight, 4,000,000), 208.7 g of medium-molecular polyisobutylene (viscosity-average molecular weight, 55,000), 208.7 g of an alicyclic saturated hydrocarbon resin, and 50.0 g of toluene. These ingredients were stirred together until the mixture became homogeneous. Thereafter, 228.2 g of an oily liquid component (isopropyl myristate) and 200.0 g of toluene were weighed out and added to the polyisobutylene solution. The resultant mixture was stirred until it became homogeneous. Thus, a coating fluid was obtained.

### (2) Application

The coating fluid was applied to the releasant-treated side of a 75 µm-thick release liner made of poly(ethylene terephthalate) (hereinafter referred to as "PET"), in such an amount as to result in a dry pressure-sensitive adhesive layer thickness of 160 µm. The coating fluid applied was dried, and the resultant pressure-sensitive adhesive layer was laminated to the PET nonwoven fabric side of a backing which was a laminate (thickness, 40 µm) of a PET film with a nonwoven fabric made of PET. Thus, a patch sheet was produced.

### (3) Preparation of Patches

### EXAMPLE 1

The patch sheet was cut into a substantially rectangular shape of 30 mm × 20 mm with a razor. The pressure-sensitive adhesive layer present in an edge part was scraped out with a pincette to produce a patch of the invention shown in Figs. 1A and 1B.

### EXAMPLES 2 AND 3

The patch sheet was cut into a substantially rectangular shape of 30 mm × 20 mm with a razor while applying the razor so that the smaller of the angles each formed by the razor and the patch sheet was an acute angle. Thus, a patch of the invention shown in Fig. 2 and a patch of the invention shown in Fig. 3 were produced in Example 2 and Example 3, respectively.

### COMPARATIVE EXAMPLE 1

The patch sheet was cut into a substantially rectangular shape of 30 mm × 20 mm with a razor while applying the razor so that the angles formed by the razor and the patch sheet were approximately right angles. Thus, a patch of Comparative Example 1 shown in Fig. 6 was produced.

### COMPARATIVE EXAMPLE 2

The patch sheet was cut into a substantially rectangular shape of 30 mm × 20 mm with a razor while applying the razor so that the angles formed by the razor and the patch sheet were approximately right angles. During the razor cutting, a near-edge part of the patch was kept being pressed with a finger. Thus, a patch of Comparative Example 2 shown in Fig. 7 was produced.

### (4) Evaluation

### Sectional Shape

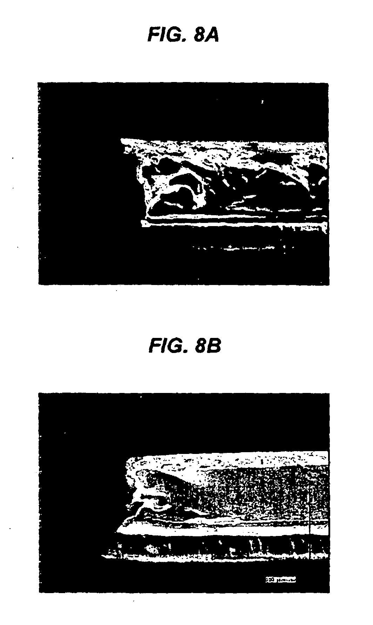

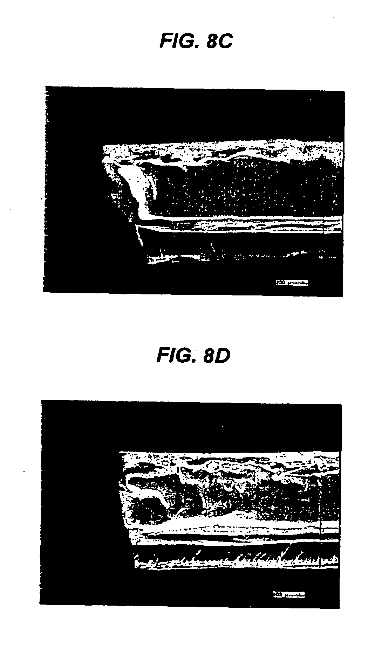

Photographs (500 diameters) of sectional shapes of the patches obtained in the Examples and Comparative Examples were taken with a digital microscope (KEYENCE, VHX-500). The result for Example 1, that for Example 2, and that for Example 3 are shown in Fig. 8A, Fig. 8B, and Fig. 8C, respectively. The result for Comparative Example 1 and that for Comparative Example 2 are shown in Fig. 8D and Fig. 8E, respectively. With respect to each image obtained, measurements were made of the distance between the edge of the backing and the position where the edge of the pressure-sensitive adhesive layer was in contact with the backing (distance 6), the distance between the edge of the release liner and the position where the edge of the pressure-sensitive adhesive layer was in contact with the release liner (distance 7), the angle formed by a plane including the surface of the backing facing the pressure-sensitive adhesive layer and a contact plane of the edge of the pressure-sensitive adhesive layer (angle 8), the angle formed by a plane including the surface of the release liner facing the pressure-sensitive adhesive layer and a contact plane of the edge of the pressure-sensitive adhesive layer (angle 9), etc. The results obtained are shown in Table 1.

**Table 1**

| | Fig. | Photograph | Distance 6 (µm) | Distance 7 (µm) | Angle 8 (degrees) | Angle 9 (degrees) |
|---|---|---|---|---|---|---|
| Example 1 | Fig. 1 | Fig. 8A | 22 | 10 | 90 | 90 |
| Example 2 | Fig. 2 | Fig. 8B | 0 | 13 | 60 | 65 |
| Example 3 | Fig. 3 | Fig. 8C | 22 | 6 | 75 | 105 |
| Comparative Example 1 | Fig.6 | Fig. 8D | 0 | 0 | 90 | 90 |
| Comparative Example 2 | Fig.7 | Fig.8E | 0 | 0 | >90 | >90 |

In the sectional view of the patch of Example 2, a nearly central part, with respect to the thickness direction, of the pressure-sensitive adhesive layer in the edge part of the patch was located on the patch center side of a segment connecting the position where the edge of the pressure-sensitive adhesive layer was in contact with the backing and the position where the edge of the pressure-sensitive adhesive layer was in contact with the release liner. Specifically, that nearly central part of the pressure-sensitive adhesive layer was apart from that segment at a distance of 35 µm as measured in the direction parallel to the backing or release liner.

### Tackiness of Edge Part

The patches of the Examples and Comparative Examples were touched by an evaluator and evaluated based on the following criteria.
A: No tackiness.
B: The edge is slightly tacky but the tackiness is allowable.
C: The edge is tacky.

### Releasability of Liner

The release liner of each of the patches of the Examples and Comparative Examples was peeled off by an evaluator, and evaluated based on the following criteria.
A: The liner can be easily peeled off.
B: The liner can be peeled off.
C: The liner is difficult to peel off.

### Protrusion of Pressure-Sensitive Adhesive Layer

A PET film (thickness, 25 µm) was superposed on each of the patches of the Examples and Comparative Examples, and a load (about 290 g-weight) was imposed thereon for 12 hours. The pressure-sensitive adhesive layer in this patch was evaluated for protrusion from the edges of the patch based on the following criteria.
A: No protrusion is observed.
B: Protrusion is observed in part of the edges, but is allowable.
C: Protrusion is observed almost throughout the whole edges.

The results obtained are shown in Table 2.

**Table 2**

| Evaluation item | Example 1 | Example 2 | Example 3 | Comparative Example 1 | Comparative Example 2 |
|---|---|---|---|---|---|
| Tackiness of edge part | A | A | B | B | C |
| Releasability of liner | A | A | A | C | C |
| Protrusion of pressure-sensitive adhesive layer | A | A | B | C | C |

### (6) Evaluation Results

In Example 3, edge tackiness and protrusion of the pressure-sensitive adhesive layer were slightly observed. Except this, Examples 1 to 3 each were free from edge tackiness and protrusion of the pressure-sensitive adhesive layer or had the tackiness and the protrusion in an allowable range. The liners of these patches could be easily peeled off.

In contrast, in Comparative Examples 1 and 2, some patches had edge tackiness, and poor liner releasability and protrusion of the pressure-sensitive adhesive layer were observed.

The following were demonstrated from the above results.
(i) When a patch at an edge part thereof has such a shape that at least a part of the edge of the pressure-sensitive adhesive layer is located on the patch center side with respect to a perpendicular segment drawn from the edge of the backing at the edge part of the patch to the release liner, then this patch is inhibited from having edge tackiness, has satisfactory release liner releasability, and is inhibited from suffering protrusion of the pressure-sensitive adhesive layer.
(ii) The shape in which the position where the edge of the pressure-sensitive adhesive layer is in contact with the backing or release liner is located on the patch center side with respect to the edge of the backing or release liner is preferred from the standpoints of edge tackiness and protrusion of the pressure-sensitive adhesive layer.
(iii) The shape in which the edge of the pressure-sensitive adhesive layer has a sectional shape that it is bent toward the center of the patch is preferred from the standpoints of edge tackiness and protrusion of the pressure-sensitive adhesive layer.

This application is based on Japanese patent application No. 2007-189250 filed July 20, 2007.

## Claims

1. A patch (10) comprising a backing (2), a pressure-sensitive adhesive layer (1), and a release liner (3),
**characterized in that**
the patch (10) at an edge part thereof has such a sectional shape that, when a perpendicular segment (5) is drawn from an edge of the backing (2) at the edge part of the patch (10) to the release liner (3), at least a part (4) of an edge of the pressure-sensitive adhesive layer (1) at the edge part of the patch (10) is located on the center side of the patch (10) with respect to the segment (5),
the edge of the pressure-sensitive adhesive layer (1) is exposed;
the thickness of the pressure-sensitive adhesive layer (1) is 10 - 700 µm; and
the thickness of the backing (2) is 10 - 200 µm.

2. The patch according to claim 1, wherein at least said part (4) of the edge of the pressure-sensitive adhesive layer (1) is located on the center side of the patch (10) with respect to either the edge of the backing (2) or an edge of the release liner (3).

3. The patch according to claim 1, wherein the edge of the pressure-sensitive adhesive layer (1) has such a sectional shape that the pressure-sensitive adhesive layer is bent toward the center of the patch (10).

4. The patch according to claim 1, wherein an edge of the release liner (3) protrudes toward the edge part side of the patch (10) from a position on the release liner where the perpendicular segment (5) drawn from the edge of the backing (2) to the release liner intersects the release liner.

5. The patch according to claim 1, wherein the pressure-sensitive adhesive layer (1) contains an organic liquid component.

6. The patch according to claim 1, which is an adhesive preparation comprising a pressure-sensitive adhesive layer (1) containing a drug.

## Patentansprüche

1. Pflaster (10), das einen Träger (2), eine druckempfindliche Klebeschicht (1) und eine Trennschicht (3) umfasst,
**dadurch gekennzeichnet, dass**
das Pflaster an einem Randteil eine Querschnittsform hat, durch die, wenn ein senkrechtes Segment (5) von einem Rand des Trägers (2) an dem Randteil des Pflasters (10) zu der Trennschicht (3) gezogen wird, sich wenigstens ein Teil (4) eines Randes der druckempfindlichen Klebeschicht (1) an dem Randteil des Pflasters (10) an der Mittelseite des Pflasters (10) in Bezug auf das Segment (5) befindet,
der Rand der druckempfindlichen Klebeschicht (1) freiliegt;
die Dicke der druckempfindlichen Klebeschicht (1) 10 - 700 µm beträgt; und
die Dicke des Trägers (2) 10 - 200 µm beträgt.

2. Pflaster nach Anspruch 1, wobei sich wenigstens der Teil (4) des Randes der druckempfindlichen Klebeschicht (1) in Bezug auf den Rand des Trägers (2) oder einen Rand der Trennschicht (3) an der Mittelseite des Pflasters (10) befindet.

3. Pflaster nach Anspruch 1, wobei der Rand der druckempfindlichen Klebeschicht (1) eine Querschnittsform hat, durch die die druckempfindliche Klebeschicht zur Mitte des Pflasters (10) hin gebogen ist.

4. Pflaster nach Anspruch 1, wobei ein Rand der Trennschicht (3) zu der Seite des Randteils des Pflasters (10) von einer Position an der Trennschicht vorsteht, an der das senkrechte Segment (5), das von dem Rand des Trägers (2) zu der Trennschicht gezogen wird, die Trennschicht schneidet.

5. Pflaster nach Anspruch 1, wobei die druckempfindliche Klebeschicht (1) eine organische flüssige Komponente enthält.

6. Pflaster nach Anspruch 1, das ein klebendes Präparat ist, das eine druckempfindliche Klebeschicht (1) umfasst, die ein Medikament enthält.

## Revendications

1. Timbre (10) comprenant un support (2), une couche adhésive autocollante (1) et une doublure de décollement (3),
**caractérisé en ce que**
le timbre (10) présente, dans une partie marginale, une forme en coupe telle que, lorsqu'un segment (5) perpendiculaire est tiré depuis un bord du support (2) à la partie marginale du timbre (10), en direction de la doublure de décollement (3), au moins une partie (4) d'un bord de la couche adhésive autocollante (1) à la partie marginale du timbre (10) se trouve du côté du centre du timbre (10) par rapport au segment (5),
le bord de la couche adhésive autocollante (1) est nu, l'épaisseur de la couche adhésive autocollante (1) est de 10-700 µm, et
l'épaisseur du support (2) est de 10-200 µm.

2. Timbre selon la revendication 1, dans lequel au moins ladite partie (4) du bord de la couche adhésive autocollante (1) se trouve du côté du centre du timbre (10) par rapport, soit au bord du support (2), soit à un bord de la doublure de décollement (3).

3. Timbre selon la revendication 1, dans lequel le bord de la couche adhésive autocollante (1) présente une forme en coupe telle que la couche adhésive autocollante est courbée en direction du centre du timbre (10).

4. Timbre selon la revendication 1, dans lequel un bord de la doublure de décollement (3) fait saillie en direction du côté de la partie marginale du timbre (10), depuis une position sur la doublure de décollement dans laquelle le segment (5) perpendiculaire tiré entre le bord du support (2) et la doublure de décollement croise la doublure de décollement.

5. Timbre selon la revendication 1, dans lequel la couche adhésive autocollante (1) contient un composant organique liquide.

6. Timbre selon la revendication 1, qui est une préparation adhésive comprenant une couche adhésive autocollante (1) contenant un médicament.
